# EUROPÄISCHE PATENTSCHRIFT

(11) **EP 3 886 822 B1**
(45) Veröffentlichungstag und Bekanntmachung des Hinweises auf die Patenterteilung: **01.01.2025**
(21) Anmeldenummer: 19813251.6
(22) Anmeldetag: 20.11.2019
(51) Int. Cl.: A61K 9/70

(54) **TRANSDERMALES SYSTEM MIT ÜBERPFLASTER UND RINGSYSTEM**
TRANSDERMAL SYSTEM HAVING A BACKING LAYER AND A RING SYSTEM
SYSTÈME TRANSDERMIQUE COMPORTANT UN PANSEMENT DE RECOUVREMENT ET UN SYSTÈME EN ANNEAU

(30) Priorität: 29.11.2018 DE 102018220589
(43) Veröffentlichungstag der Anmeldung: 06.10.2021
(73) Patentinhaber: LTS Lohmann Therapie-Systeme AG, 56626 Andernach (DE)
(72) Erfinder: HAMMES, Florian, 56626 Andernach (DE); TOMELERI, Anja, 56567 Neuwied (DE); KLEUDGEN, Tobias, 56729 Ettringen (DE)
(74) Vertreter: Schweitzer, Klaus
(86) Internationale Anmeldenummer: PCT/EP2019/081912
(87) Internationale Veröffentlichungsnummer: WO 2020/109103

(56) Entgegenhaltungen:
- EP-A1- 2 921 184
- EP-A1- 3 067 050
- WO-A1-2009/147387

## Beschreibung

Die vorliegende Erfindung betrifft ein transdermales System zur Verwendung in klinischen und präklinischen Studien, die Verwendung einer zylinderförmigen Trennschicht in diesen Studien, sowie ein Verfahren zur Ermittlung von pharmakologischen und physikalischen Daten. Weiterhin betrifft die vorliegende Erfindung ein System zur Verbesserung der Haftung eines Transdermalen Therapeutischen Systems (TTS), die Verwendung dieses Systems sowie einen Kit, der dieses System beinhaltet.

Für die Entwicklung von neuen transdermalen therapeutischen Systemen (TTS) ist es notwendig, diese in präklinischen und klinischen Studien zu untersuchen. Im Rahmen von präklinischen und klinischen Studien kommt es zu Problemen, wenn die applizierten wirkstoffhaltigen TTS schlecht oder nur unvollständig auf der Haut haften. Bei den applizierten TTS handelt es sich um vollständige transdermale therapeutische Systemen, bei denen jedoch unter Umständen die Haftelemente noch nicht optimiert wurden. Außerdem werden die ersten Studien im Regelfall an Minischweinen durchgeführt. Die Beschaffenheit der Haut von Minischweinen ist der Beschaffenheit der menschlichen Haut sehr ähnlich, jedoch wird die Klebkraft eines TTS durch die Borsten der Minischweine beeinträchtigt. Weiterhin stellt die starke mechanische Beanspruchung bei Studien mit Minischweinen eine Herausforderung für die Klebkraft eines TTS dar. Daher sind die transdermal therapeutischen Systeme nicht hinsichtlich der Haftung auf der Haut der Minischweine optimiert. Bei längerer Applikationsdauer undloder schwierigen Applikationsstellen undloder mechanischer Beanspruchung können sich die TTS im ungünstigen Fall komplett von der Haut lösen und abfallen.

Auch bei kommerziell erhältlichen TTS gibt es Systeme, die beispielsweise aufgrund einer langen Tragedauer einer erhöhten Gefahr der vorzeitigen Ablösung des TTS von der menschlichen Haut unterliegen.

Um ein vorzeitiges Ablösen zu verhindern, wird für Systeme, die keine ausreichende Haftung aufweisen, beispielsweise in der DE 102009022915 A1 die Verwendung eines Überpflasters beschrieben. Ein solches Überpflaster führt einerseits zu einer besseren Haftung des TTS auf der Haut und schützt das TTS andererseits vor mechanischer Beanspruchung.

Bei der Verwendung solcher Überpflaster tritt oftmals das Problem auf, dass sich das transdermale therapeutische System (TTS) und das Überpflaster nach Beendigung der Applikation nicht mehr voneinander trennen lassen. Bei präklinischen und klinischen Studien ist es notwendig, dass das TTS nach Beendigung der Applikation analytisch ausgewertet wird. Das Überpflaster beeinflusst diese analytische Auswertung oder macht sie gar unmöglich. Des Weiteren besteht die Gefahr, dass der Wirkstoff aus dem TTS über die Ränder der zum TTS gehörigen rückseitigen Abdeckung in die Klebeschicht des Überpflasters migriert.

Die Aufgabe der vorliegenden Erfindung war es, ein System bereitzustellen, bei dem es nicht zu einer vorzeitigen Ablösung des TTS kommt und bei dem es nicht zu einer Migration des Wirkstoffs über die Ränder der zum TTS gehörigen rückseitigen Abdeckung in das Überpflaster kommt.

Gelöst wir diese Aufgabe durch ein System umfassend:
(i) ein transdermales therapeutisches System (TTS) mit einer der Haut zugewandten und einer der Haut abgewandten Seite;
(ii) eine zylinderförmige Trennschicht mit einem Außenumfang, einem Innenumfang, einer Stirnfläche und einer Bodenfläche, wobei die zylinderförmige Trennschicht auf die der Haut abgewandten Seite des TTS aufgebracht ist und der Außenumfang der zylinderförmigen Trennschicht das TTS vollumfänglich überragt und das TTS den Innenumfang der zylinderförmigen Trennschicht vollumfänglich überragt;
(iii) ein Überpflaster, das auf die zylinderförmige Trennschicht aufgebracht ist und diese vollumfänglich überragt.

Erfindungsgemäß überragt der Außenumfang der zylinderförmigen Trennschicht das TTS vollumfänglich und das TTS überragt vollumfänglich den Innenumfang der zylinderförmigen Trennschicht. Das bedeutet, dass das TTS und die zylinderförmige Trennschicht überlappen. Das TTS ist ein flächiges System, das einen Außenumfang besitzt, der größer ist als der Innenumfang der zylinderförmigen Trennschicht, aber kleiner ist als der Außenumfang der zylinderförmigen Trennschicht. Da der Außenumfang der zylinderförmigen Trennschicht größer ist als der Außenumfang des TTS, legt sich die zylinderförmige Trennschicht über die Ränder der zum TTS gehörigen rückseitigen Abdeckung. Dadurch kommen die Ränder des TTS nicht mit dem Überpflaster in Kontakt.

Damit es zu keiner Migration des Wirkstoffs über die Ränder der zum TTS gehörigen rückseitigen Abdeckung in das Überpflaster kommt, ist es bevorzugt, dass die zylinderförmige Trennschicht wirkstoffundurchlässig ist.

Hinsichtlich der Größe des Innenumfangs der zylinderförmigen Trennschicht gibt es keine spezifischen Limitierungen, vorausgesetzt der Innenumfang der zylinderförmigen Trennschicht ist kleiner als der Außenumfang des TTS, so dass das TTS den Innenumfang der zylinderförmigen Trennschicht vollumfänglich überragt. Der Innenumfang der Trennschicht schließt eine Fläche ein, deren Flächeninhalt bevorzugt von 0,1 bis 30,0 cm², besonders bevorzugt von 1,0 bis 15,0 cm², ganz besonders bevorzugt von 5,0 bis 10,0 cm² ist.

Da es sich bei der zylinderförmigen Trennschicht um eine Schicht handelt, die einen Innenumfang besitzt, kann das Überpflaster mit dem TTS innerhalb der Fläche, die von dem Innenumfang der zylinderförmigen Trennschicht gebildet wird in Kontakt treten. Wenn das Überpflaster und das TTS über die Fläche, die der Innenumfang der zylinderförmigen Trennschicht bildet in Kontakt treten, haftet das TTS an dem Überpflaster. Dadurch ist es möglich, das TTS besser zu fixieren und es verrutscht nicht unter dem Überpflaster.

Weiterhin weist die zylinderförmige Trennschicht bevorzugt eine Dicke von 5 bis 20 µm, besonders bevorzugt von 8 bis 17 µm, ganz besonders bevorzugt von 10 bis 15 µm auf.

Hinsichtlich des Bereichs, in dem das TTS den Innenumfang der zylinderförmigen Trennschicht überragt, gibt es keine spezifischen Limitierungen. Bevorzugt überragt das TTS den Innenumfang der zylinderförmigen Trennschicht um mindestens 1 mm, besonders bevorzugt mindestens 3 mm, ganz besonders bevorzugt mindestens 5 mm vollumfänglich.

Weiterhin ist es bevorzugt, dass der Außenumfang der zylinderförmigen Trennschicht das TTS um mindestens 1 mm, besonders bevorzugt mindestens 3 mm, ganz besonders bevorzugt mindestens 5 mm vollumfänglich überragt.

Hinsichtlich der Form der zylinderförmigen Trennschicht gibt es keine spezifischen Limitierungen, vorausgesetzt, dass die zylinderförmige Trennschicht einen Außenumfang, einen Innenumfang, eine Stirnfläche und eine Bodenfläche aufweist. Der Außenumfang und der Innenumfang der zylinderförmigen Trennschicht besitzen unabhängig voneinander eine kreisrunde, ovale oder polygonale Form. Besitzt die Trennschicht eine polygonale Form, so ist es bevorzugt, dass die Ecken abgerundet sind.

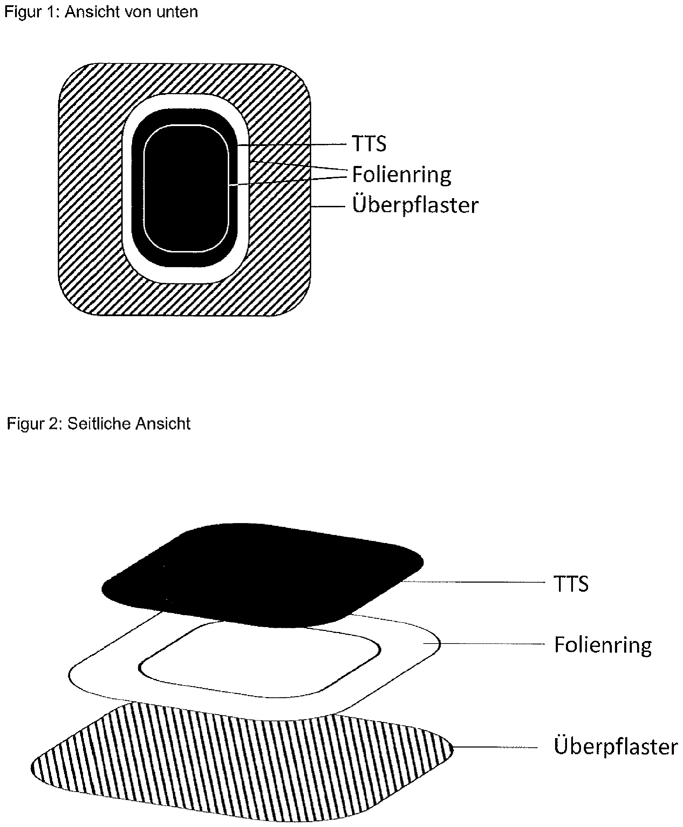

Der Außenumfang und der Innenumfang können eine identische oder unterschiedliche Form besitzen. Bevorzugt besitzen der Außenumfang und der Innenumfang eine identische Form. Besonders bevorzugt besitzen der Außenumfang und der Innenumfang der zylinderförmigen Trennschicht eine runde oder ovale Form. Bevorzugt ist, dass der Außenumfang der Trennschicht der Außenform des eingesetzten TTS entspricht und die Außenform der Trennschicht die Außenform des TTS überragt
Figur 1 zeigt eine Ansicht des erfindungsgemäßen Systems von unten, das heißt von der Hautseite aus.
Figur 2 zeigt eine seitliche Ansicht des erfindungsgemäßen Systems.

Das erfindungsgemäße System umfasst ein TTS (1), einen Folienring (2) und ein Überpflaster (3), wobei der Folienring die zylinderförmige Trennschicht darstellt.

Hinsichtlich des Materials, aus dem die zylinderförmige Trennschicht aufgebaut ist, gibt es keine spezifischen Limitierungen. Die zylinderförmige Trennschicht kann klebend oder nicht klebend ausgestaltet sein. Bevorzugt besteht die zylinderförmige Trennschicht aus einem Material, das aus der Gruppe ausgewählt ist, die Polyethylenterephthalat, weichgemachte Vinylacetat-Vinylchlorid-Copolymerisate, Nylon, Ethylen-Vinylacetat-Copolymerisate, weichgemachtes Polyvinylchlorid, Polyurethan, Polyvinylidenchlorid, Polypropylen, Polyethylen oder Polyamid umfasst.

Das Überpflaster besteht bevorzugt aus einem textilen Gewebe. Das Gewebe kann in einer oder zwei Richtungen elastisch ausgestaltet sein. Damit das Überpflaster sicher auf der Haut und auf dem eigentlichen TTS fixiert werden kann, ist dieses mit einer haftklebenden Schicht ausgestattet.

Geeignete Grundpolymere für eine haftklebende Schicht sind beispielsweise in Tan, Pfister, PSTT Vol. 2, No.2 Februar 1999, Seiten 60 - 69 beschrieben. Geeignete Grundpolymere sind beispielsweise Polyacrylate, Poly(meth)acrylate, Polyacrylsäure, Cellulose-Derivate, insbesondere Methyl- und Ethylcellulosen, Polyisobutylen, Ethylen-Vinylacetat-Copolymere, natürliche und synthetische Kautschuke wie Styrol-Dien-Copolymere, Styrol-Butadien-Blockcopolymere, Styrol-Isopren-Blockcopolymere, Acrylnitril-Butadien-Kautschuk, Butylkautschuk oder Neoprenkautschuk, sowie Heißschmelzkleber. Als Haftkleber kommen auch solche auf Silikonbasis in Betracht. Mit Vorteil können auch geeignete Mischungen der genannten Polymere oder Hybridkleber aus Acrylat-Monomeren und Silikon-Monomeren zum Einsatz kommen.

Erfindungsgemäß enthält die haftklebende Schicht, mit der das Überpflaster ausgerüstet ist, als Grundpolymere Polymere, welche vorzugsweise aus der Polyacrylate, Poly(meth)acrylate, Polyacrylsäure, Cellulose-Derivate, Polyisobutylen, Ethylen-Vinylacetat-Copolymere, natürliche und synthetische Kautschuke wie Styrol-Dien-Copolymere, Styrol-Butadien-Blockcopolymere, Styrol-Isopren-Blockcopolymere, Acrylnitril-Butadien-Kautschuk, Butylkautschuk oder Neoprenkautschuk, sowie Heißschmelzkleber und Haftkleber auf Silikonbasis umfassenden Gruppe ausgewählt sind.

Bei dem TTS, das in dem erfindungsgemäßen System verwendet wird, handelt es sich um ein vollständiges TTS, das
(a) einen Wirkstoffträger oder ein Wirkstoffreservoir und
(b) eine Rückschicht umfasst.

Die Rückschicht eines TTS muss für den im TTS enthaltenen Wirkstoff undurchlässig sein, um ein unerwünschtes Austreten des Wirkstoffs aus der Seite des TTS, die der Haut abgewandt ist, zu verhindern. Hierfür werden spezielle Kunststofffolien, z.B. metallisierte Kunststofffolien eingesetzt. Bevorzugt werden aluminierte Folien aus Polyethylenterephthalat eingesetzt. Der Vorteil beim Verwenden einer metallisierten Folie liegt darin, dass sie undurchlässig für nahezu alle pharmazeutischen Wirkstoffe und Licht ist, was gerade bei lichtempfindlichen Wirkstoffen den Vorteil eines zuverlässigen Lichtschutzes bietet.

Als Materialien für die wirkstoffundurchlässige Rückschicht des TTS eignen sich vor allem Polyester, die sich durch besondere Festigkeit auszeichnen, wie z. B. Polyethylenterephthalat und Polybutylenterephthalat, darüber hinaus aber nahezu beliebige andere hautverträgliche Kunststoffe, wie Polyvinylchlorid, Polyurethan, Polyvinylidenchlorid, Ethylen-Vinylacetat-Copolymerisate, Polyvinylacetat, Vinylacetat-Vinylchlorid-Copolymerisate, Nylon, Polyethylen, Polypropylen, Polyurethane, Polyamid, Cellulosederivate und viele andere mehr. Bevorzugt sind Polyethylenterephthalat, weichgemachte Vinylacetat-Vinylchlorid-Copolymerisate, Nylon, Ethylen-Vinylacetat-Copolymerisate, weichgemachtes Polyvinylchlorid, Polyurethan, Polyvinylidenchlorid, Polypropylen, Polyethylen und Polyamid.

Bei dem erfindungsgemäßen System ist die zylinderförmige Trennschicht auf der Rückschicht des TTS aufgebracht.

Wenn das erfindungsgemäße System nach Beendigung der Applikation analytisch ausgewertet werden soll, ist die zylinderförmige Trennschicht bevorzugt nicht klebend. Ein solches erfindungsgemäßes System zeichnet sich dadurch aus, dass das TTS, die zylinderförmige Trennschicht und das Überpflaster voneinander trennbar sind. Dadurch wird die analytische Auswertung nach der Applikation erleichtert, da die analytischen Daten nicht durch das Vorhandensein des Überpflasters verfälscht werden.

Weiterhin betrifft die vorliegende Erfindung die Verwendung einer nicht klebenden, zylinderförmigen Trennschicht mit einem Außenumfang, einem Innenumfang, einer Stirnfläche und einer Bodenfläche zur Ermittlung von pharmakologischen und physikalischen Daten in klinischen und präklinischen Studien.

Die vorliegende Erfindung betrifft weiterhin ein Verfahren zur Ermittlung von pharmakologischen und physikalischen Daten umfassend:
(a) Bereitstellung eines erfindungsgemäßen Systems umfassend:
   (a1) Aufbringen einer nicht klebenden, zylinderförmige Trennschicht mit einem Außenumfang, einem Innenumfang, einer Stirnfläche und einer Bodenfläche auf ein TTS mit einer der Haut zugewandten und einer der Haut abgewandten Seite, so dass die nicht klebende, zylinderförmige Trennschicht auf die der Haut abgewandten Seite des TTS aufgebracht ist und der Außenumfang der nicht klebenden, zylinderförmigen Trennschicht dass TTS vollumfänglich überragt und das TTS den Innenumfang der nicht klebenden, zylinderförmigen Trennschicht vollumfänglich überragt;
   (a2) Aufbringen eines Überpflasters auf die nicht klebende, zylinderförmige Trennschicht, so dass das Überpflaster die nicht klebende, zylinderförmige Trennschicht vollumfänglich überragt;
(b) Aufbringen des Systems erhalten aus (a) auf Haut;
(c) Erhebung von pharmakologischen und physikalischen Daten.

Weiterhin betrifft die vorliegende Erfindung ein System zur Verbesserung der Haftung eines TTS umfassend:
(ii) eine zylinderförmige Trennschicht mit einem Außenumfang, einem Innenumfang, einer Stirnfläche und einer Bodenfläche;
(iii) ein Überpflaster, das auf die zylinderförmige Trennschicht aufgebracht ist und diese vollumfänglich überragt.

Die zylinderförmige Trennschicht (ii) und das Überpflaster (iii) sind wie zuvor beschrieben beschaffen.

Auch betrifft die vorliegende Erfindung die Verwendung eines Systems umfassend:
(ii) eine zylinderförmige Trennschicht mit einem Außenumfang, einem Innenumfang, einer Stirnfläche und einer Bodenfläche;
(iii) ein Überpflaster, das auf die zylinderförmige Trennschicht aufgebracht ist und diese vollumfänglich überragt;
zur Verbesserung der Haftung eines TTS undloder zur Verhinderung der Migration des Wirkstoffs über die Ränder des TTS.

Ein solches System wird zusammen mit einem kommerziell erhältlichen TTS verwendet, wobei die zylinderförmige Trennschicht auf die der Haut abgewandten Seite des TTS aufgebracht wird und der Außenumfang der zylinderförmigen Trennschicht das TTS vollumfänglich überragt und das TTS den Innenumfang der zylinderförmigen Trennschicht vollumfänglich überragt.

Hinsichtlich des in dem TTS verwendeten Wirkstoffs gibt es keine spezifischen Einschränkungen, vorausgesetzt der Wirkstoff ist für die Verabreichung mittels eines TTS geeignet. Ein Beispiel für einen solchen Wirkstoff ist Buprenorphin. Beispiele für kommerziell erhältliche Buprenorphin haltige TTS sind Transtec^{®}, Norspan^{®} und Butrans^{®}.

Wie bereits ausgeführt wurde, tritt das Überpflaster mit dem TTS innerhalb der Fläche, die von dem Innenumfang der zylinderförmigen Trennschicht gebildet wird, in Kontakt und das TTS haftet an dem Überpflaster. Dadurch ist es möglich, ein kommerziell erhältliches TTS zu fixieren und somit seine Haftung auf der Haut zu verbessern. Auch tritt bei einem solchen System keine Migration des Wirkstoffs über die Ränder des TTS auf, da der Außenumfang der zylinderförmigen Trennschicht das TTS vollumfänglich überragt.

Weiterhin betrifft die vorliegende Erfindung einen Kit umfassend:
ein transdermales therapeutisches System (TTS) und ein erfindungsgemäßes System zur Verbesserung der Haftung eines TTS umfassend:
(ii) eine zylinderförmige Trennschicht mit einem Außenumfang, einem Innenumfang, einer Stirnfläche und einer Bodenfläche;
(iii) ein Überpflaster, das auf die zylinderförmige Trennschicht aufgebracht ist und diese vollumfänglich überragt.

## Patentansprüche

1. System umfassend:
(i) ein transdermales therapeutisches System (TTS) mit einer der Haut zugewandten und einer der Haut abgewandten Seite;
(ii) eine zylinderförmige Trennschicht mit einem Außenumfang, einem Innenumfang, einer Stirnfläche und einer Bodenfläche, wobei die zylinderförmige Trennschicht auf die der Haut abgewandten Seite des TTS aufgebracht ist und der Außenumfang der zylinderförmigen Trennschicht dass TTS vollumfänglich überragt und das TTS den Innenumfang der zylinderförmigen Trennschicht vollumfänglich überragt;
(iii) ein Überpflaster, das auf die zylinderförmige Trennschicht aufgebracht ist und diese vollumfänglich überragt, wobei die zylinderförmige Trennschicht wirkstoffundurchlässig ist, und das Überpflaster mit einer haftklebenden Schicht zu Fixierung auf der Haut ausgerüstet ist.

2. System nach Anspruch 1, **dadurch gekennzeichnet, dass** der Innenumfang der zylinderförmigen Trennschicht eine Fläche einschließt, deren Flächeninhalt von 0,1 bis 30,0 cm², bevorzugt von 1,0 bis 15,0 cm², besonders bevorzugt von 5,0 bis 10,0 cm² ist und die zylinderförmige Trennschicht eine Dicke von 5 bis 20 µm, bevorzugt 8 bis 17 µm, besonders bevorzugt von 10 bis 15 µm aufweist.

3. System nach Anspruch 1 oder 2, **dadurch gekennzeichnet, dass** das TTS den Innenumfang der zylinderförmigen Trennschicht um mindestens 1 mm, bevorzugt mindestens 3 mm, besonders bevorzugt mindestens 5 mm vollumfänglich überragt und der Außenumfang der zylinderförmigen Trennschicht das TTS um mindestens 1 mm, bevorzugt mindestens 3 mm, besonders bevorzugt mindestens 5 mm vollumfänglich überragt und wobei der Außenumfang und der Innenumfang der zylinderförmigen Trennschicht unabhängig voneinander eine kreisrunde, oval oder polygonale Form besitzen.

4. System nach einem oder mehreren der Ansprüche 1 bis 3, **dadurch gekennzeichnet, dass** die zylinderförmige Trennschicht aus einem Material besteht, das aus der Gruppe ausgewählt ist, die Polyethylenterephthalat, weichgemachte Vinylacetat-Vinylchlorid-Copolymerisate, Nylon, Ethylen-Vinylacetat-Copolymerisate, weichgemachtes Polyvinylchlorid, Polyurethan, Polyvinylidenchlorid, Polypropylen, Polyethylen oder Polyamid umfasst.

5. System nach einem oder mehreren der Ansprüche 1 bis 4, **dadurch gekennzeichnet, dass** die haftklebende Schicht als Grundpolymere Polymere enthält, welche vorzugsweise aus der Polyacrylate, Poly(meth)acrylate, Polyacrylsäure, Cellulose-Derivate, Polyisobutylen, Ethylen-Vinylacetat-Copolymere, natürliche und synthetische Kautschuke wie Styrol-Dien-Copolymere, Styrol-Butadien-Blockcopolymere, Styrol-Isopren-Blockcopolymere, Acrylnitril-Butadien-Kautschuk, Butylkautschuk oder Neoprenkautschuk, sowie Heißschmelzkleber und Haftkleber auf Silikonbasis umfassenden Gruppe ausgewählt sind.

6. System nach einem oder mehreren der Ansprüche 1 bis 5, **dadurch gekennzeichnet, dass** das TTS
(a) einen Wirkstoffträger oder ein Wirkstoffreservoir und
(b) eine Rückschicht umfasst.

7. System nach Anspruch 6, **dadurch gekennzeichnet, dass** die zylinderförmige Trennschicht auf der Rückschicht des TTS aufgebracht ist.

8. System nach einem oder mehreren der Ansprüche 1 bis 7, **dadurch gekennzeichnet, dass** die zylinderförmige Trennschicht nicht klebend ist und das TTS, die zylinderförmige Trennschicht und das Überpflaster voneinander trennbar sind.

9. Verfahren zur Ermittlung von pharmakologischen und physikalischen Daten umfassend:
(a) Bereitstellung eines Systems nach einem oder mehreren der Ansprüche 1 bis 8, wobei die Trennschicht eine nicht klebende, zylinderförmige Trennschicht ist, umfassend:
(a1) Aufbringen einer nicht klebenden, zylinderförmige Trennschicht mit einem Außenumfang, einem Innenumfang, einer Stirnfläche und einer Bodenfläche auf eine TTS mit einer der Haut zugewandten und einer der Haut abgewandten Seite, so dass die nicht klebende, zylinderförmige Trennschicht auf die der Haut abgewandten Seite des TTS aufgebracht ist und der Außenumfang der nicht klebenden, zylinderförmigen Trennschicht dass TTS vollumfänglich überragt und das TTS den Innenumfang der nicht klebenden, zylinderförmigen Trennschicht vollumfänglich überragt;
(a2) Aufbringen eines Überpflasters auf die nicht klebende, zylinderförmige Trennschicht, so dass das Überpflaster die nicht klebende, zylinderförmige Trennschicht vollumfänglich überragt;
(b) Aufbringen des Systems erhalten aus (a) auf Haut;
(c) Erhebung von pharmakologischen und physikalischen Daten.

10. System zur Verbesserung der Haftung eines TTS umfassend:
(ii) eine zylinderförmige Trennschicht mit einem Außenumfang, einem Innenumfang, einer Stirnfläche und einer Bodenfläche;
(iii) ein Überpflaster, das auf die zylinderförmige Trennschicht aufgebracht ist und diese vollumfänglich überragt.

11. Kit umfassend:
ein transdermales therapeutisches System (TTS) und
ein System nach Anspruch 10.

12. Verwendung eines Systems nach Anspruch 10 zur Verbesserung der Haftung eines TTS undloder zur Verhinderung der Migration des Wirkstoffs über die Ränder des TTS.

## Claims

1. System comprising:
(i) a transdermal therapeutic system (TTS) with a side facing towards the skin and a side facing away from the skin;
(ii) a cylindrical release layer with an outer circumference, an inner circumference, a front face and a bottom face, wherein the cylindrical release layer is applied to the side of the TTS facing away from the skin, and the outer circumference of the cylindrical release layer protrudes fully circumferentially over the TTS, and the TTS protrudes fully circumferentially over the inner circumference of the cylindrical release layer;
(iii) an overplaster which is applied to the cylindrical release layer and protrudes fully circumferentially over the latter, the cylindrical release layer being impermeable to active substance, and the overplaster being equipped with a pressure-sensitive adhesive layer for fixing to the skin.

2. System according to Claim 1, **characterized in that** the inner circumference of the cylindrical release layer encloses an area whose area content is from 0.1 to 30.0 cm², preferably from 1.0 to 15.0 cm², particularly preferably from 5.0 to 10.0 cm², and the cylindrical release layer has a thickness of 5 to 20 µm,preferably of 8 to 17 µm, particularly preferably of 10 to 15 µm.

3. System according to Claim 1 or 2, **characterized in that** the TTS protrudes fully circumferentially over the inner circumference of the cylindrical release layer by at least 1 mm, preferably by at least 3 mm, particularly preferably by at least 5 mm, and the outer circumference of the cylindrical release layer protrudes fully circumferentially over the TTS by at least 1 mm, preferably by at least 3 mm, particularly preferably by at least 5 mm, and wherein the outer circumference and the inner circumference of the cylindrical release layer have, independently of each other, a circular, oval or polygonal shape.

4. System according to one or more of Claims 1 to 3, **characterized in that** the cylindrical release layer is made of a material selected from the group comprising polyethylene terephthalate, plasticized vinyl acetate-vinyl chloride copolymers, nylon, ethylene-vinyl acetate copolymers, plasticized polyvinyl chloride, polyurethane, polyvinylidene chloride, polypropylene, polyethylene or polyamide.

5. System according to one or more of Claims 1 to 4, **characterized in that** the pressure-sensitive adhesive layer contains, as base polymer, polymers preferably selected from the group comprising polyacrylates, poly(meth)acrylates, polyacrylic acid, cellulose derivatives, polyisobutylene, ethylene-vinyl acetate copolymers, natural and synthetic rubbers such as styrene-diene copolymers, styrene-butadiene block copolymers, styrene-isoprene block copolymers, acrylonitrile butadiene rubber, butyl rubber or neoprene rubber, and also hot-melt adhesives and pressure-sensitive adhesives based on silicone.

6. System according to one or more of Claims 1 to 5, **characterized in that** the TTS comprises
(a) an active substance carrier or an active substance reservoir and
(b) a backing layer.

7. System according to Claim 6, **characterized in that** the cylindrical release layer is applied to the backing layer of the TTS.

8. System according to one or more of Claims 1 to 7, **characterized in that** the cylindrical release layer is not adhesive, and the TTS, the cylindrical release layer and the overplaster are separable from one another.

9. Method for the determination of pharmacological and physical data, comprising:
(a) making available a system according to one or more of Claims 1 to 8, wherein the release layer is a non-adhesive, cylindrical release layer, comprising:
(a1) applying a non-adhesive, cylindrical release layer with an outer circumference, an inner circumference, a front face and a bottom face to a TTS with a side facing towards the skin and a side facing away from the skin, such that the non-adhesive, cylindrical release layer is applied to the side of the TTS facing away from the skin, and the outer circumference of the non-adhesive, cylindrical release layer protrudes fully circumferentially over the TTS, and the TTS protrudes fully circumferentially over the inner circumference of the non-adhesive, cylindrical release layer;
(a2) applying an overplaster to the non-adhesive, cylindrical release layer, such that the overplaster protrudes fully circumferentially over the non-adhesive, cylindrical release layer;
(b) applying the system obtained from (a) to skin;
(c) collecting pharmacological and physical data.

10. System for improving the adhesion of a TTS, comprising:
(ii) a cylindrical release layer with an outer circumference, an inner circumference, a front face and a bottom face;
(iii) an overplaster which is applied to the cylindrical release layer and protrudes fully circumferentially over the latter.

11. Kit comprising:
a transdermal therapeutic system (TTS) and
a system according to Claim 10.

12. Use of a system according to Claim 10 for improving the adhesion of a TTS and/or for preventing the migration of the active substance over the edges of the TTS.

## Revendications

1. Système comprenant :
(i) un système thérapeutique transdermique (TTS) avec un côté tourné vers la peau et un côté opposé à la peau ;
(ii) une couche de séparation cylindrique avec une circonférence extérieure, une circonférence intérieure, une face frontale et une face inférieure, la couche de séparation cylindrique étant appliquée sur le côté du TTS opposé à la peau et la circonférence extérieure de la couche de séparation cylindrique dépassant entièrement le TTS et le TTS dépassant entièrement la circonférence intérieure de la couche de séparation cylindrique ;
(iii) un sur-pansement appliqué sur la couche de séparation cylindrique et la dépassant entièrement, la couche de séparation cylindrique étant imperméable aux substances actives, et le sur-pansement étant équipé d'une couche adhésive sensible à la pression pour la fixation sur la peau.

2. Système selon la revendication 1, **caractérisé en ce que** la circonférence intérieure de la couche de séparation cylindrique inclut une surface dont la superficie est de 0,1 à 30,0 cm², de préférence de 1,0 à 15,0 cm², particulièrement de préférence de 5,0 à 10,0 cm², et la couche de séparation cylindrique a une épaisseur de 5 à 20 µm, de préférence de 8 à 17 µm, particulièrement de préférence de 10 à 15 µm.

3. Système selon la revendication 1 ou 2, **caractérisé en ce que** le TTS dépasse entièrement la circonférence intérieure de la couche de séparation cylindrique d'au moins 1 mm, de préférence d'au moins 3 mm, particulièrement de préférence d'au moins 5 mm, et la circonférence extérieure de la couche de séparation cylindrique dépasse entièrement le TTS d'au moins 1 mm, de préférence d'au moins 3 mm, particulièrement de préférence d'au moins 5 mm, et dans lequel la circonférence extérieure et la circonférence intérieure de la couche de séparation cylindrique ont indépendamment une forme circulaire, ovale ou polygonale.

4. Système selon une ou plusieurs des revendications 1 à 3, **caractérisé en ce que** la couche de séparation cylindrique est faite d'un matériau choisi parmi le groupe comprenant le polyéthylène téréphtalate, les copolymères plastifiés d'acétate de vinyle et de chlorure de vinyle, le nylon, les copolymères d'éthylène et d'acétate de vinyle, le polychlorure de vinylidène plastifié, le polyuréthane, le polychlorure de vinylidène, le polypropylène, le polyéthylène ou le polyamide.

5. Système selon une ou plusieurs des revendications 1 à 4, **caractérisé en ce que** la couche adhésive sensible à la pression contient comme polymères de base des polymères choisis de préférence parmi le groupe comprenant les polyacrylates, les poly(méth)acrylates, l'acide polyacry-lique, les dérivés de cellulose, le polyisobutylène, les copolymères d'éthylène-acétate de vinyle, les caoutchoucs naturels et synthétiques tels que les copolymères de styrène-diène, les copolymères blocs de styrène-butadiène, les copolymères blocs de styrène-isoprène, le caoutchouc acrylonitrile-butadiène, le caoutchouc butyle ou le caoutchouc néoprène, ainsi que les adhésifs thermo-fusibles et les adhésifs à base de silicone.

6. Système selon une ou plusieurs des revendications 1 à 5, **caractérisé en ce que** le TTS comprend
(a) un support de substance active ou un réservoir de substance active et
(b) une couche de support.

7. Système selon la revendication 6, **caractérisé en ce que** la couche de séparation cylindrique est appliquée sur la couche de support du TTS.

8. Système selon une ou plusieurs des revendications 1 à 7, **caractérisé en ce que** la couche de séparation cylindrique n'est pas adhésive et **en ce que** le TTS, la couche de séparation cylindrique et le sur-pansement sont séparables les uns des autres.

9. Procédé pour déterminer des données pharmacologiques et physiques comprenant :
(a) fourniture d'un système selon une ou plusieurs des revendications 1 à 8, dans lequel la couche de séparation est une couche de séparation cylindrique non adhésive, comprenant :
(a1) application d'une couche de séparation cylindrique non adhésive avec une circonférence extérieure, une circonférence intérieure, une face frontale et une face inférieure sur un TTS avec un côté tourné vers la peau et un côté opposé à la peau, de sorte que la couche de séparation cylindrique non adhésive soit appliquée sur le côté du TTS opposé à la peau et que la circonférence extérieure de la couche de séparation cylindrique non adhésive dépasse entièrement le TTS et que le TTS dépasse entièrement la circonférence intérieure de la couche de séparation cylindrique non adhésive ;
(a2) application d'un sur-pansement sur la couche de séparation cylindrique non adhésive, de sorte que le sur-pansement dépasse entièrement la couche de séparation cylindrique non adhésive ;
(b) application du système obtenu à partir de (a) sur la peau ;
(c) collecte de données pharmacologiques et physiques.

10. Système pour améliorer l'adhérence d'un TTS comprenant :
(ii) une couche de séparation cylindrique avec une circonférence extérieure, une circonférence intérieure, une face frontale et une face inférieure ;
(iii) un sur-pansement appliqué sur la couche de séparation cylindrique et la dépassant entièrement.

11. Kit comprenant :
un système thérapeutique transdermique (TTS) et
un système selon la revendication 10.

12. Utilisation d'un système selon la revendication 10 pour améliorer l'adhérence d'un TTS et/ou pour empêcher la migration de la substance active au-delà des bords du TTS.
